# EUROPEAN PATENT APPLICATION

(11) **EP 3 181 084 A1**
(43) Date of publication of application: **21.06.2017**
(21) Application number: 17150807.0
(22) Date of filing: 12.11.2004
(51) Int. Cl.: A61B 18/14, A61B 18/18

(54) **LENGTH ADJUSTABLE ELECTRO-SURGICAL PENCIL WITH SUCTION MEANS**

(30) Priority: 14.11.2003 DK 200301695
(62) Divisional of application: 07122453.9
(71) Applicant: Lina Medical ApS, 2600 Glostrup (DK)
(72) Inventor: Kornerup, Niels, 2960 Rungsted (DK); Busch-Madsen, Patrick, 3550 Slangerup (DK)
(74) Representative: Inspicos P/S

(57) **Abstract**

An electro-surgical (ES) pencil (1;21) is provided of the kind comprising an elongated housing (2;22), an electrode (5) for cutting and/or coagulating, a suction tube (3), and a suction hose connecting the suction tube with a suction unit. The pencil comprises adjustment means and an electrical wire for providing electrical power to the pencil is placed inside the suction hose.

## Description

### Field of Invention

The present invention relates to an Electro-Surgical (ES) pencil apparatus comprising an elongated housing, an electrode for cutting and/or coagulation arranged with its longitudinal axis parallel to the longitudinal axis of said housing, a suction tube arranged with its longitudinal axis parallel to the longitudinal axis of said housing e.g. offset from the longitudinal axis of the electrode, and a suction hose connecting the suction tube with a suction unit.

### Background of the Invention

Electro-Surgical (ES) pencils are used in surgery, typically for cutting tissue and/or for coagulating blood. An ES pencil usually comprises a hand-piece into which electrodes of many different shapes and sizes may be placed. The electrode is supplied with a high frequency, typically Radio Frequency (RF), alternating current signal by an RF source. The RF source is typically called the Electro-Surgical Unit or ESU for short. By varying the voltage and/or the frequency of the RF signal supplied by the ESU the electrode can be used in different modes. Two common modes are a cutting mode and a coagulating mode.

During a surgical procedure it is often desired to change the length of the electrode used. Therefore electrodes come in different lengths. However, it is both expensive and time consuming to change electrodes when a change in length is desired during surgery. Expensive since more electrodes are consumed and time consuming because the surgical procedure needs to be stopped while the electrode is being changed. While using an ES pencil, smoke is often generated which makes visibility more difficult for the surgeon as well as being unpleasant for the surgeon and surgical staff. Therefore smoke evacuation shrouds can be attached to an ES pencil, which suck the smoke away via a suction device. Smoke shrouds are available either as an integrated part of the ES pencil or as a separate shroud attached to the ES pencil.

### Description of the Prior Art

US patent number 5,234,428 discloses an electro-surgical pencil with integrated suction means for smoke evacuation. The electrode is placed in the centre of the inlet of the suction means. The disadvantage with this is that since the electrode is placed in the centre of the suction tube the efficiency of the smoke evacuation is reduced. Also the electrode in this case is of fixed length, which makes it rather expensive and time consuming to change lengths.

Another, more advanced ES pencil is disclosed by US patent number 5,693,044. This ES pencil also has the electrode arranged in the centre of the suction tube. However, this ES pencil has an arrangement for changing the length of the electrode via a telescopic action. This means that the electrode doesn't need to be changed in order to change the length. In addition, a second ES pencil is disclosed which has an argon gas coagulator in addition to the electrode and smoke evacuation means. This pencil is quite complex since there are a lot of components packaged into its housing, all centred in the suction tube. Another disadvantage with the disclosed ES pencil is that the distance between the tip of the electrode and the tip of the suction tube is not easily or widely adjustable.

A disadvantage of both of the above-mentioned ES pencils is that since the electrode and the electronics are arranged inside the suction tube, fluids sucked into the ES pencil via the suction means come into contact with the electrode and the electronics. This can lead to short circuits as well as contamination of the electrode and electronics.

British GB 2 380 679 A discloses an electro-surgical pencil using different interchangeable electrode blades of fixed length and an aspirator tube slidable in an axial direction in the housing to position the free end of the tube in relation to electrode blades. When the length of the electrode blade must be changed a new blade must be inserted.

A further electro-surgical pencil is described in US patent number 4,919,129. This electro-surgical pencil also allows the electro-surgical pencil to be extended with the addition of extension pieces. However, these extension pieces extend both the suction means and the electrode. The electrode is furthermore adjustable with respect to the tip of the suction means. However, as with other known ES pencils including the one known from GB 2 380 679, it is difficult and time consuming to exchange the extension pieces during a surgical procedure and there is a risk of such extension pieces or blades unintentionally fall off and drop down into the surgical site or wound.

Other known electro-surgical pencils, such as the ones described in US 5,413,575, US 5,055,100, US 3,906,955, US 5,318,565, US 5,071,418, and US 4,307,720 allow the tip of the electrode to be displaced with respect to the tip of the suction means. However, none of these pencils allow the surgeon to quickly change the effective length of the electro-surgical pencil.

### Summary of the Present Invention

A first aspect of the present invention is to provide an ES pencil as mentioned in the opening paragraph having suction means, which are more efficient than currently known.

A second aspect of the present invention is to provide an ES pencil as mentioned in the opening paragraph where the effective or "active" length of the ES pencil can be easily adjusted.

A third aspect of the present invention is to provide an ES pencil as mentioned in the opening paragraph where extra devices, such as e.g. lights, coagulation devices, irrigation devices, etc., can be added to the device in order to match the ES pencil to the needs of the surgeon.

A fourth aspect of the present invention is to provide an ES pencil as mentioned in the opening paragraph where the suction means can be effectively used for both gasses and fluids.

The new and unique way in which the present invention fulfills the above-mentioned aspects is by providing an ES pencil as mentioned in the opening paragraph wherein an electrical wire for providing electrical power to the pencil is placed inside the hose.

The advantage of placing the wire inside the tube is that it is a low cost and simple method of packaging the tube and wire together so that they are less likely to tangle. In addition, with the wire inside the tube, the wire is less likely to catch on something.

In a preferred embodiment, the electronic components are placed outside the suction tube. This allows the suction means to work with both fluids and gasses.

The pencil can further comprise adjustment means arranged such that the tip of the suction tube and the tip of the electrode are displaceable along the housing's longitudinal axis with respect to the location of the surgeon's hand on the electro-surgical pencil. This configuration gives rise to a number of benefits.

One benefit of this configuration is that the suction tube and the electrode can be arranged as independently adjustable elements within the housing. This results in an ES pencil, which can be used in many different situations. In addition, it is possible to adjust the distance between the tip of the electrode and the tip of the suction tube over a large interval. The ES pencil can also be adjusted such that the tip of the electrode is either ahead of the tip of the suction tube or behind the tip of the suction tube. In cases where the electrode is predominately being used the tip of the electrode can be placed ahead of the tip of the suction tube. In cases where the suction tube is predominately being used the tip of the electrode can be placed behind the tip of the suction tube.

Furthermore, since the tip of the suction tube and the tip of the electrode are both displaceable with respect to the location where the surgeon holds the ES pencil, the effective or "active" length of the ES pencil is easily and quickly adjusted. The effective length can be changed either by displacing the electrode and the suction tube with respect to the housing or by the surgeon changing the location where he or she holds the ES pencil.

Another benefit of this configuration is that the flow through the suction tube is more effective since the electrode is placed external to the suction tube. Because the suction means are more effective, the suction means can be made smaller which results in better visibility for the surgeon.

A further benefit of this configuration is that the ES pencil can be held in different ways depending on its desired use. When the surgeon is using the electrode for cutting or coagulating, the surgeon can hold the ES pencil with the suction tube above the electrode. In this case the lowest point of the ES pencil during surgery is the electrode. This ensures that the suction tube doesn't get in the way during surgery. In addition, since smoke rises, placing the suction tube above the electrode when cutting or coagulating increases the efficiency of the smoke evacuation. When the surgeon is using the suction means, the surgeon can hold the ESU pencil with the suction tube below the electrode. In this position, the suction tube is the lowest point and therefore ensures that the electrode doesn't get in the way.

The side-by-side configuration of the suction tube and electrode is also beneficial since extra devices can easily be placed inside the suction tube. Some examples are, a light source, an argon gas coagulator, irrigation means, etc. This makes the device easily expandable and customisable.

In a preferred embodiment, the means of adjusting the length between the tip of the electrode and the tip of the suction tube could comprise at least one complementary rail and groove arrangement. A rail and groove arrangement gives easy adjustability as well as a simple construction. By rail and groove is meant any arrangement, which permits movement along an axis. Some examples are, a tube sliding in a circular channel, a first tube sliding in a second tube of slightly larger diameter, a slot and tab arrangement, etc.

In an advantageous embodiment, a first complementary rail and groove arrangement is arranged between a carrier of the electrode and the housing. One example of such an embodiment would be grooves cut in opposite sides of the housing, and complementary rails formed on opposite sides of the carrier, which fit into the grooves arranged into the housing. This is a simple and low cost solution, which gives a large adjustment range.

If it is desired to allow the suction tube and the electrode to move as a single unit, a second complementary rail and groove arrangement can be arranged between the suction tube and the carrier.

In another embodiment, a complementary rail and groove arrangement can be arranged between the housing and the suction tube. In this way, the position of the suction tube and the electrode can be adjusted independently.

In order to lock the position of the tip of the electrode and/or the suction tube with respect to the housing, at least one locking means can be provided to lock the electrode and/or the suction tube in position relative to the housing. Locking means allow the cutting electrode and/or the suction tube to be able to exert force without changing length. The locking means can take many forms, some examples are a friction lock, a latch mechanism, a clamping mechanism, etc.

One advantageous embodiment of a locking mechanism can be a ratchet mechanism with a release mechanism arranged between the carrier and the housing. The ratchet mechanism would permit the electrode to be pulled out of the housing, but would prevent the electrode from being pushed into the housing. The release mechanism would be used to release the ratchet mechanism such that the electrode could be pushed into the housing. This is an advantageous embodiment, because the forces acting on the electrode are typically acting to push the electrode back into the housing. By ratchet mechanism it should be understood any mechanism, which permits movement in one direction and prevents movement in the opposite direction. In a preferred embodiment, the position of the suction tube can be locked in position relative to the electrode. This allows the electrode and suction tube to be extended as a single unit. This is useful since the suction means will stay at the same distance from the tip of the electrode during extension and retraction of the electrode.

### Brief Description of the Figures

The invention will be explained in greater detail below where further advantageous properties and example embodiments are described with reference to the drawings, in which
Fig. 1 is a representation of a first embodiment of an ES pencil according to the invention seen in perspective in a first position,
Fig. 2 shows the same ESU pencil in a second position,
Fig. 3 shows the same ESU pencil in a third position,
Fig. 4 shows in a perspective section view the same ESU pencil,
Fig. 5 shows an exploded perspective section view of the same ESU pencil
Fig. 6 shows a perspective view of a second embodiment of an ES pencil according to the invention seen in a first position,
Fig. 7 shows a perspective view of a second embodiment of an ES pencil according to the invention seen in a second position, and
Fig. 8 shows an exploded perspective view of a second embodiment of an ES pencil according to the invention.

### Description of a Preferred Embodiment of the Invention

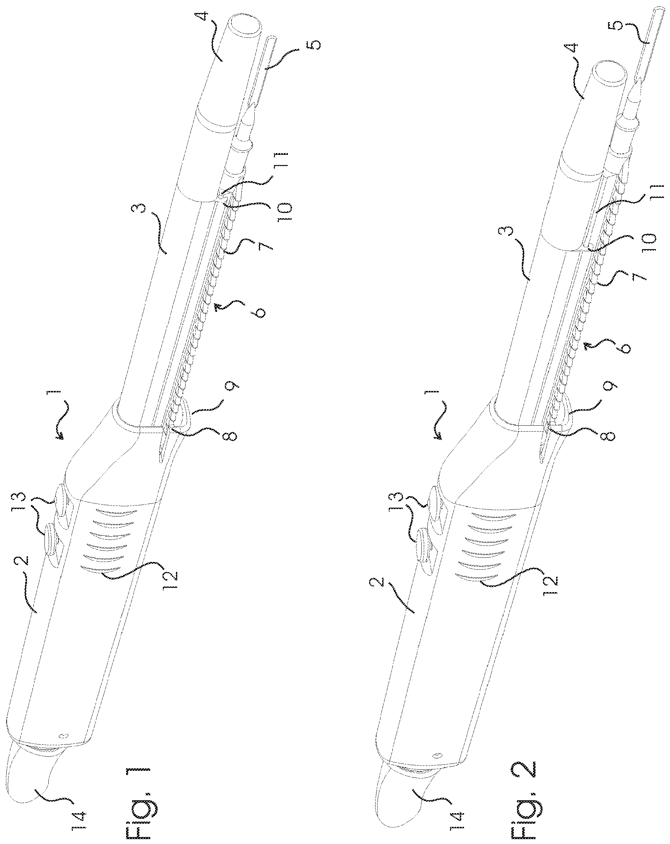

Fig. 1-5 show a first embodiment 1 of an Electro Surgical (ES) pencil according to the current invention. The embodiment shown in the figures should be used as an example only and should not limit the scope of the invention in any way.

The ESU pencil comprises a housing 2, a suction tube 3 with a suction nozzle 4, and an electrode 5 for cutting and/or coagulation. The electrode 5 is mounted to a carrier 6. The carrier 6 is arranged so that it can slide in and out of the housing 2. This can be thought of as a rail and groove arrangement, where the carrier 6 is the rail and the housing 2 is the groove.

A first set of teeth 7 placed on the carrier 6 form a ratchet mechanism together with a second set of teeth 8 placed on a flexible member 9 of the housing 2. Due to the shape of the first set of teeth 7, the carrier 6 can be pulled out of the housing 2 but is prevented from being pushed into the housing 2. By depressing the flexible member 9 of the housing 2, the second set of teeth 8 disengage from the first set of teeth 7 and the carrier 6 can be pushed into the housing 2.

The suction tube 3 is provided with a groove 10, which matches to a rail 11 formed on the carrier 6. This allows the suction tube 3 to be displaced relative to the carrier 6. This displacement can be seen by comparing figures 1 and 2. In the position shown in Fig. 1, the tip of the suction nozzle 4 is a bit in front of the tip of the electrode 5. In fig. 2, the tip of the suction nozzle 4 is behind the tip of the electrode 5.

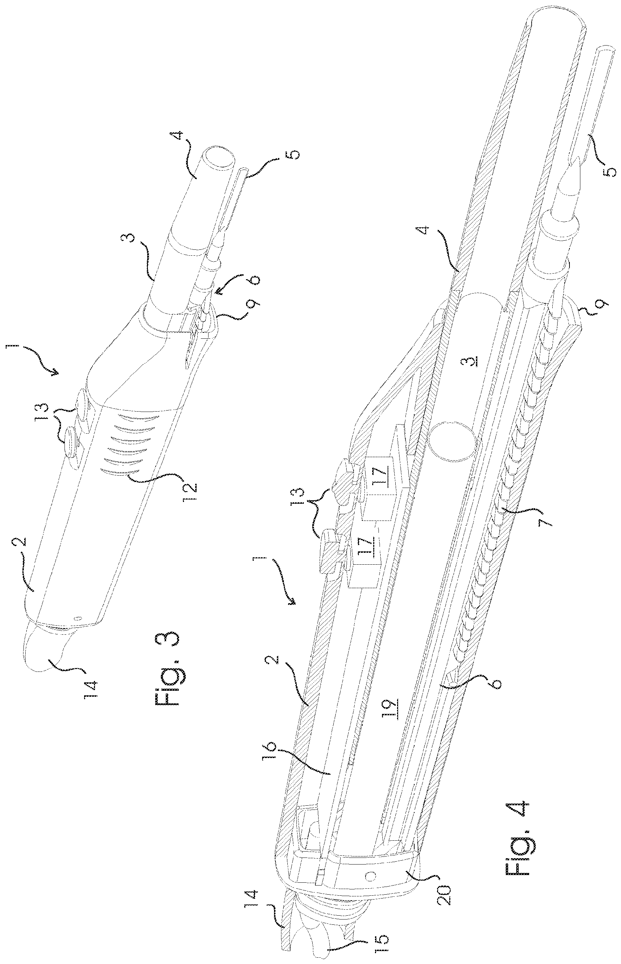

In addition to adjusting the distance between the tip of the electrode 5 and the tip of the suction nozzle 4, the distance between the housing and the tip of the electrode 5 can also be adjusted. This adjustment can be seen by comparing figures 1 and 2. In fig. 1 the suction nozzle 4 and the electrode 5 have been pulled out of the housing, which results in a long ES pencil. In fig. 3, the suction nozzle 4 and the electrode 5 have been pushed into the housing 2. In this way, the length of the ES pencil 1 is reduced.

In this way, the ES pencil 1 shown, can be adjusted to any length between a minimum length where the carrier 6 and the suction tube 3 are fully retracted in the housing 2 and a maximum length where the carrier 6 and the suction tube 3 are fully extracted from the housing 2. In addition, at any of these positions, the position of the suction nozzle 4 can be adjusted relative to the position of the electrode 5 by sliding the suction tube 3 forwards or backwards on the carrier 6.

In the case where the ES pencil 1 is used for suction, the suction nozzle 4 is preferably extracted so that it is level or in front of the electrode 5. In this case, the surgeon could hold the ES pencil upside down so that the suction nozzle 4 is below the electrode 5. This makes it easier for the surgeon to manipulate the ES pencil without worrying that the electrode 5 gets in the way.

In the case where the ES pencil 1 is used for cutting and/or coagulation, the suction nozzle 4 is preferably retracted a bit so that the suction nozzle 4 does not block the surgeon's view of the electrode 5. In this case the surgeon would most likely hold the ES pencil 1 in the position shown in the figures, with the suction nozzle 4 above the electrode 5. In this orientation, the suction nozzle 4 is not likely to get in the way of the electrode 5.

In the embodiment shown in figures 1 to 5, the suction tube 3 is held in position by friction present in the rail and groove arrangement between the carrier 6 and the suction tube 3. The friction is a type of simple locking mechanism. This simple locking mechanism, friction, is possible since there is not so much force present on the tip of the suction tube 3. If more force is exerted on the tip of the suction tube 3 than is expected, the suction tube 3 will be pushed into the housing 2. Other forms of locking mechanisms could also be implemented, some examples being a latch mechanism, a clamp mechanism, a ratchet mechanism, etc...

The housing 2 has a set of ridges 12 to increase the surgeon's grip on the ES pencil 1. The housing 2 is equipped with two buttons 13 to control the operation of the ES pencil. The buttons 13 are waterproof so that fluids splashed onto the housing do not interfere with the electrical operation of the ESU pencil. The location of the buttons 13 in this embodiment fixes the location where the surgeon holds the ES pencil. The surgeon can't change his or her hand position without loosing the ability to operate the buttons.

One end of a suction hose 14 is attached to the rear of the housing 2. The other end of the suction hose 14 is connected to a suction unit (not shown). The electrical power for the ES pencil 1 is provided via an electrical wire 15 (hidden inside the suction hose 14) connected to an Electro Surgical Unit (ESU, not shown). Placing the wire 15 inside the suction hose 14 combines the wire 15 and suction hose 14 into a single unit, preventing tangles between the wire 15 and the suction hose 14. This makes the ES pencil 1 easier to handle. The suction unit can either be a part of the ESU or it could be a separate unit.

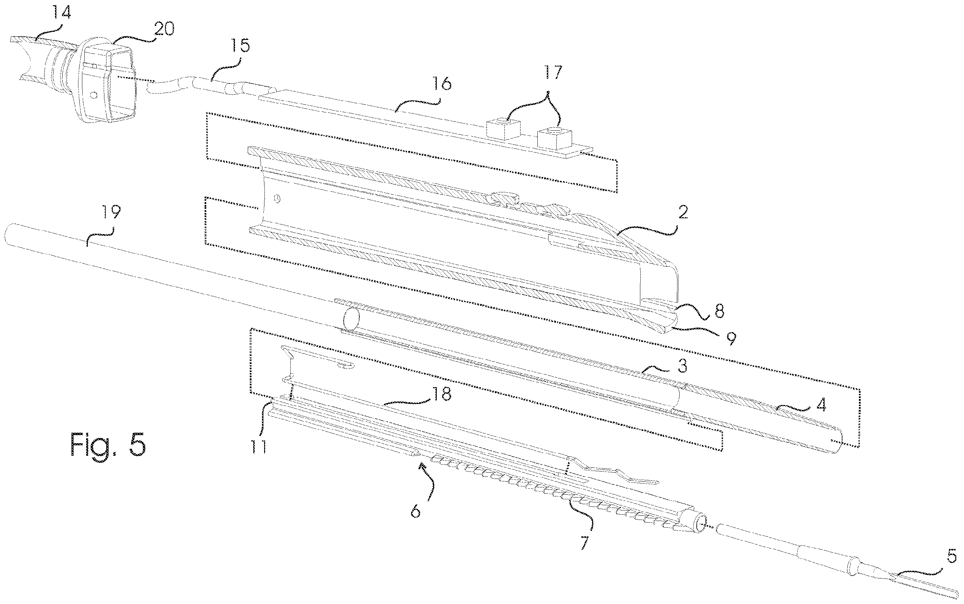

Fig. 4 shows a 3D section view of the ES pencil 1. Fig. 5 shows an exploded view of the 3D section of the ES pencil 1 shown in fig. 4.

Fig. 4 and 5 show how the ESU pencil 1 is assembled. The electrical power gets supplied to the ES pencil via the electrical wire 15. The electrical wire 15 is connected to a circuit board 16. The buttons 13 on the housing are connected to the circuit board 16 via switches 17. The electrical power is supplied from the circuit board 15 to the electrode via a wire clip 18 which is inserted in the carrier 6 and passes around the suction tube 3 making contact with the bottom of the circuit board 16 which is plated with an electrically conducting surface (not shown). Since the connection between the wire clip 18 and the circuit board 15 can slide while remaining in electrical connection with each other, the carrier 6 can be retracted and extended relative to the housing 2 without breaking the electrical connection.

In order for the suction tube 3 to extend and retract relative to the housing 2, the suction tube 3 is arranged in a telescopic manner with an inner suction tube 19. The inner suction tube 19 is fixed in position relative to the housing 2. The suction tube 3 has a slightly larger diameter than the inner suction tube 19 allowing the two suction tubes to slide relative to each other while maintaining a relatively good air seal. The electrode 5 can be removed from the carrier 6 and electrodes of other shapes inserted in the carrier. The suction hose 14 and the inner part of the suction tube 19 are connected to the housing via an end cap 20.

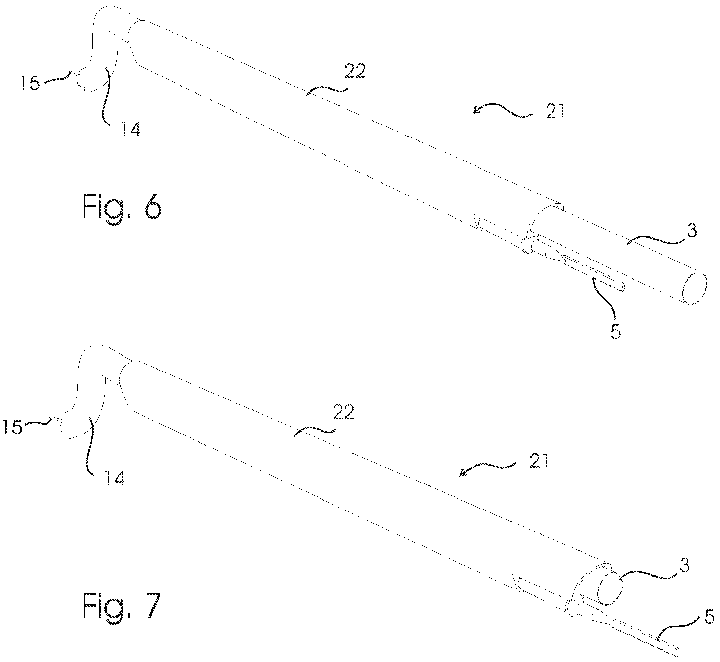

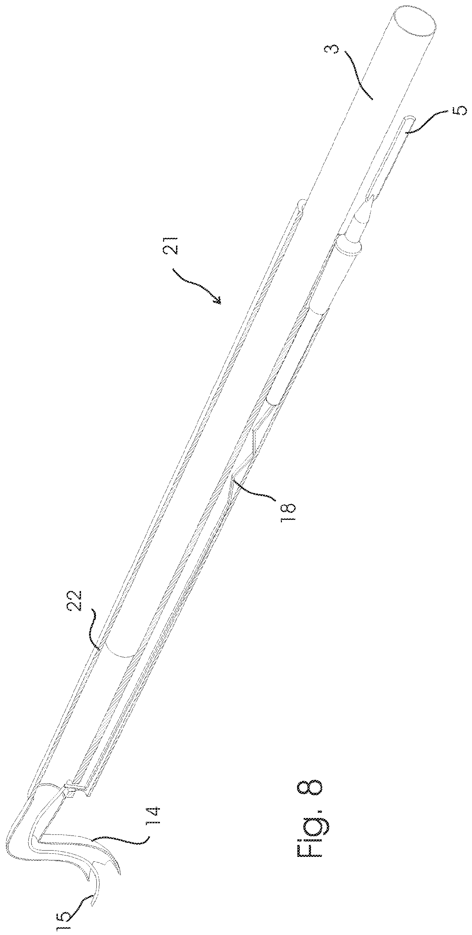

A lower cost embodiment 21 of an ES pencil according to the invention is shown in figures 6-8. In the embodiment shown, there are no buttons on the housing 22 of the ES pencil 21. In this embodiment the surgeon controls the operation of the ES pencil via a foot switch (not shown). Due to the foot switch controller, it is not important where the surgeon holds the ES pencil 21 since there are no buttons. Therefore the surgeon can change the effective or "active" length of the ES pencil 21 by shifting the location or place where he or she holds the ES pencil 21. For example if a long ES pencil is desired, the surgeon can hold the ES pencil further back on the housing. If a shorter ES pencil is desired, the surgeon can hold the ES pencil further forward on the housing.

In this embodiment 21, the electrode 5 can be arranged in a fixed position relative to the housing 22, and only the suction tube 3 is arranged to be displaceable. In the embodiment shown, the suction tube 3 slides in the housing 22. This can again be thought of as a simple rail and groove arrangement where the suction tube 3 is the rail and the housing 22 is the groove. Another option (not shown) is that the suction tube 3 is fixed in position, and the electrode 5 is displaceable.

As in the previous embodiment, the electrical power for the ES pencil is supplied by a wire 15, which in this case has been placed inside the suction hose 14.

As mentioned in the introductory section, any number of additional devices can be placed inside the suction tube. Some examples of devices, which could be placed inside the suction tube are, a light, an argon coagulator unit, an irrigator, etc.

In addition, it would also be possible to place an argon coagulator around the electrode. Since the suction means are not centred about the electrode the argon gas is not sucked away by the suction means before it has a chance to perform its function.

### Numbered Embodiments

1. An electro-surgical pencil of the kind comprising
   - an elongated housing,
   - an electrode for cutting and/or coagulating arranged with its longitudinal axis parallel to the longitudinal axis of said housing,
   - a suction tube arranged with its longitudinal axis parallel to the longitudinal axis of said housing and offset from the longitudinal axis of the electrode, and
   - a suction hose connecting the suction tube with a suction unit,
   characterized in, that the pencil comprises adjustment means and that an electrical wire for providing electrical power to the pencil is placed inside the suction hose.
2. The electro-surgical pencil according to embodiment 1 comprising a number of electronic components characterised in, that said components are placed outside the suction tube.
3. The electro-surgical pencil according to embodiment 2, characterised in, that the adjustment means are arranged such that the tip of the suction tube and the tip of the electrode are displaceable along the housing's longitudinal axis with respect to the location of the surgeon's hand on the electro-surgical pencil.
4. The electro-surgical pencil according to embodiment 3 characterized in, that the adjustment means comprise at least one complementary rail and groove arrangement.
5. The electro-surgical pencil according to embodiment 2 characterized in, that a first complementary rail and groove arrangement is arranged between a carrier of the electrode and the housing.
6. An electro-surgical pencil according to embodiment 3 characterized in, that a second complementary rail and groove arrangement is arranged between the suction tube and the carrier of the electrode.
7. An electro-surgical pencil according to embodiment 3 characterized in, that a third complementary rail and groove arrangement is arranged between the suction tube and the housing.
8. An electro-surgical pencil according to embodiment 1 - 7 characterized in, that at least one locking means is provided to lock the position of the electrode and/or the suction tube relative to the housing.
9. An electro-surgical pencil according to embodiment 4 - 8 characterized in, that a ratchet mechanism with a release mechanism is provided, said ratchet mechanism arranged between the carrier and the housing in such a way as to permit the carrier to be pulled out of the housing but prevent the carrier from being pushed into the housing, and said release mechanism arranged in such a way as to release the ratchet mechanism to permit the carrier to be pushed into the housing.
10. An electro-surgical pencil according to embodiment 4 - 9 characterized in, that second locking means are provided to lock the position of the suction tube relative to the electrode.
11. An electro-surgical pencil of the kind comprising
   - an elongated housing (2),
   - an electrode (5) for cutting and/or coagulating arranged with its longitudinal axis parallel to the longitudinal axis of said housing,
   - a suction tube (3) arranged with its longitudinal axis parallel to the longitudinal axis of said housing and offset from the longitudinal axis of the electrode, and
   - a suction hose (14) connecting the suction tube with a suction unit,
   characterized in, that an electrical wire for providing electrical power to the pencil is placed inside the suction hose and that the pencil comprises adjustment means (6,7,8,9,10,11), said adjustment means are arranged such that the tip of the suction tube (3) and the tip of the electrode (5) are displaceable along the housing's (2)longitudinal axis with respect to the location of the surgeon's hand on the electro-surgical pencil.

## Claims

1. An electro-surgical pencil (1;21) of the kind comprising
- an elongated housing (2),
- an electrode (5) for cutting and/or coagulating arranged with its longitudinal axis parallel to the longitudinal axis of said housing (2),
- a suction tube (3) arranged with its longitudinal axis parallel to the longitudinal axis of said housing (2), and
- a suction hose connecting the suction tube with a suction unit,
**characterized in, that** the pencil comprises adjustment means and that an electrical wire for providing electrical power to the pencil is placed inside the suction hose.

2. The electro-surgical pencil according to claim 1 comprising a number of electronic components **characterised in, that** said components are placed outside the suction tube.

3. The electro-surgical pencil (1;21) according to claim 1 or 2, **characterised in, that** the adjustment means are arranged such that a tip of the suction tube and a tip of the electrode are displaceable along the housing's longitudinal axis with respect to the location of the surgeon's hand on the electro-surgical pencil.

4. The electro-surgical pencil according to claim 3 **characterized in, that** the adjustment means allows adjustment such that the tip of the electrode is either ahead of the tip of the suction tube or behind the tip of the suction tube.

5. The electro-surgical pencil according to claim 3 or 4 **characterized in, that** the adjustment means comprise at least one complementary rail and groove arrangement.

6. The electro-surgical pencil according to claim 5 **characterized in, that** a first complementary rail and groove arrangement is arranged between a carrier of the electrode and the housing.

7. An electro-surgical pencil according to any of claims 5-6, **characterized in, that** a second complementary rail and groove arrangement is arranged between the suction tube and the carrier of the electrode.

8. An electro-surgical pencil according to any of claims 5-7, **characterized in, that** a third complementary rail and groove arrangement is arranged between the suction tube and the housing.

9. An electro-surgical pencil according to claim 1 - 8 **characterized in, that** at least one locking means is provided to lock the position of the electrode and/or the suction tube relative to the housing, the locking means being a friction lock.

10. An electro-surgical pencil according to any of the precedings **characterized in, that** the suction tube and the electrode are independently adjustable elements within the housing.

11. An electro-surgical pencil according to claims 1 - 10 **characterized in, that** second locking means are provided to lock the position of the suction tube relative to the electrode.

12. The electro-surgical pencil according to any of claims 5-11, **characterized in that** the rail and groove arrangement forms a tube sliding in a circular channel.

13. The electro-surgical pencil according to any of the preceding claims, **characterized in that** the suction tube and the electrode are allowed to move as a single unit.

14. The electro-surgical pencil according to claim 6-12 and 13, wherein the suction tube and electrode are allowed to move as a single unit by means of a second complementary rail and groove arrangement arranged between the suction tube and the carrier.

15. The electro-surgical pencil according to any of the preceding claims, **characterized in that** the longitudinal axis of said suction tube is offset from the longitudinal axis of the electrode.
